# EUROPÄISCHE PATENTANMELDUNG

(11) **EP 0 910 989 A1**
(43) Veröffentlichungstag der Anmeldung: **28.04.1999**
(21) Anmeldenummer: 98118813.9
(22) Anmeldetag: 05.10.1998
(51) Int. Cl.: A61B 6/08, A61B 6/03

(54) **Röntgeneinrichtung**

(30) Priorität: 17.10.1997 DE 19746096
(71) Anmelder: SIEMENS AKTIENGESELLSCHAFT, 80333 München (DE)
(72) Erfinder: Schütz, Oliver Dr., 91052 Erlangen (DE)

(57) **Zusammenfassung**

Röntgeneinrichtung aufweisend (α) ein Röntgengerät (2) mit einem (eine Röntgenstrahlenquelle (10) und eine Röntgenstrahlenempfangseinrichtung (11)) aufweisenden Röntgensystem, welches (zur Aufnahme von 2D-Projektionen aus unterschiedlichen Projektionswinkeln von einem Bereich eines zu untersuchenden Objektes (P) mit anschließender 3D-Bildrekonstruktion des Bereiches des Objektes (P)) verstellbar ist, und ein (β) Positionserfassungssystem mit wenigstens einer (dem Röntgensystem zugeordneten) Kamera (15,16,20 bis 23), (welche den Bewegungen des Röntgensystems folgt), und mit (in bezug auf die Kamera (15,16,20 bis 23) ortsfesten, von dem Röntgensystem getrennt) angeordneten Mitteln (17,24), auf die die Kamera (15,16,20 bis 23) (zur Ermittlung der Positionen der Röntgenstrahlenquel le (10), der Röntgenstrahlenempfangseinrichtung (11) und zur Ermittlung der unterschiedlichen Projektionswinkel der 2D-Projektionen für die 3D-Bildrekonstruktion) gerichtet ist, wobei die Kamera (15,16,20 bis 23) und die (17,24) Mittel (außerhalb des von der Röntgenstrahlenquelle (10) ausgehenden Röntgenstrahlenbündels) angeordnet sind.

## Beschreibung

Die Erfindung betrifft eine Röntgeneinrichtung aufweisend ein Röntgengerät mit einem eine Röntgenstrahlenquelle und eine Röntgenstrahlenempfangseinrichtung aufweisenden Röntgensystem, welches zur Aufnahme von 2D-Projektionen aus unterschiedlichen Projektionswinkeln von einem Bereich eines zu untersuchenden Objektes mit anschließender 3D-Bildrekonstruktion des Bereiches des Objektes verstellbar ist, und eine Vorrichtung zur Ermittlung der Projektionsgeometrien.

Röntgengeräte der eingangs genannten Art weisen zur Aufnahme der Röntgenstrahlenquelle und der Röntgenstrahlenempfangseinrichtung in der Regel einen C-Bogen auf, welcher derart an dem Röntgengerät in einer Halterung gelagert ist, daß er längs seines Umfanges in einem bestimmten Winkelbereich motorisch verstellbar ist (Orbitalbewegung). Zur Gewinnung von 2D-Projektionen aus unterschiedlichen Projektionswinkeln für die 3D-Bildrekonstruktion, beispielsweise eines Körperbereiches eines Lebewesens, mit Hilfe des C-Bogen-Röntgengerätes, wird der C-Bogen nach entsprechender Plazierung relativ zu dem zu untersuchenden Lebewesen bei der Aufnahme der 2D-Projektionen von dem Körperbereich des Lebewesens längs seines Umfanges verstellt. Aus den während der Verstellbewegung des C-Bogens mit dem Röntgensystem aufgenommenen 2D-Projektionen werden anschließend 3D-Bilder des Körperbereiches des Lebewesens rekonstruiert. Die 3D-Bildrekonstruktion setzt allerdings die genaue Kenntnis der Projektionsgeometrie, d. h. die Kenntnis der Position der Röntgenstrahlenquelle und der Röntgenstrahlenempfangseinrichtung sowie der Projektionswinkel während der Aufnahme jeder der einzelnen 2D-Projektionen voraus.

Als problematisch erweist es sich, daß bekannte stationäre und ganz besonders mobile C-Bogen-Röntgengeräte mechanische Instabilitäten, insbesondere die Verstellung des C-Bogens längs seines Umfanges betreffend, aufweisen, wodurch Abweichungen der realen Verstellbewegung des C-Bogens von der idealen Verstellbewegung auftreten, welche die Bestimmung der Projektionsgeometrien erschweren.

Zur Bestimmung der Projektionsgeometrien sind folgende zwei Methoden bekannt:
a) Aus der DE 195 12 819 A1 ist die Verwendung eines in der Regel aus Plexiglas mit eingesetzten Metallstrukturen gebildeten Markerrings bekannt, welcher um den zu untersuchenden Körperbereich des Lebewesens angeordnet wird. In den 2D-Projektionen des zu untersuchenden Körperbereiches sind die Metallstrukturen des Markerrings sichtbar, so daß aus deren Position die jeweiligen Projektionsgeometrien der 2D-Projektionen berechenbar sind. Dieses Verfahren hat jedoch den Nachteil, daß der Markerring einen relativ großen Durchmesser aufweist, so daß der Abstand zwischen Röntgenstrahlenquelle und Markerring bei C-Bögen kleinen Durchmessers sehr klein ist (wenige Zentimeter). Die Metallstrukturen werden daher mit sehr großer Vergrößerung in den 2D-Projektionen abgebildet, so daß große Teile der 2D-Projektionen von den Metallstrukturen überlagert sind. Des weiteren wird nur ein kleiner Bereich der Metallstrukturen des Markerrings in den 2D-Projektionen abgebildet, so daß die Bestimmung der Projektionsgeometrien anhand der geringen Anzahl von abgebildeten Metallstrukturen schwierig ist.
b) Eichmessungen vor der eigentlichen Patientenmessung unter der Annahme, daß das Systemverhalten, d. h. im wesentlichen die Verstellbewegungen des C-Bogens in hohem Maße reproduzierbar ist. Dieses Verfahren ist jedoch sehr zeitaufwendig und zudem nur bei mechanisch verstärkten stationären C-Bogen-Röntgengeräten anwendbar. Die Anwendung auf mobile Röntgengeräte ist wegen der bereits erwähnten mechanischen Instabilität derartiger Röntgengeräte nicht möglich, wobei mechanische Stabilisierungen aufgrund der großen, die Mobilität einschränkenden Gewichtszunahme für mobile Röntgengeräte ausgeschlossen sind.

Aus der US 5,109,397 ist ein mobil ausgeführter Computertomograph bekannt, dessem um ein Rotationszentrum rotierenden eine Röntgenstrahlenquelle und eine Röntgenstrahlenempfangseinrichtung aufweisenden Röntgensystem Sensoren zugeordnet sind, welche sich mit dem Röntgensystem mitbewegen und zur Detektion seitlicher Bewegungen des Röntgensystems während eines Scans mit einem um das Rotationszentrum angeordneten, ortsfesten Ring zusammenwirken. Die Sensoren erzeugen dabei Signale, deren Auswertung die Ermittlung der Abstände zwischen ihrem definierten Anbringungsort und dem Ring gestatten. Die gewonnenen Daten werden anschließend zur Rekonstruktion von Schnittbildern herangezogen. Der Ring ist dabei im Ausbreitungsweg eines von der Röntgenstrahlenquelle ausgehenden Röntgenstrahlenbündels angeordnet.

Sensoren zur Ermittlung des Abstandes zweier Objekte voneinander sind auch aus der DE 43 32 254 C1 und der DE 94 08 562 U1 bekannt. Die Ermittlung des Abstandes erfolgt dabei durch die Laufzeitmessung von Schallwellen oder elektromagnetischen Wellen.

In der GB 1 569 885 ist ein Computertomograph mit einem um ein Rotationszentrum rotierenden Röntgensystem beschrieben, welcher Mittel zur Bestimmung der Projektionsgeometrien des Röntgensystems während eines Scans aufweist. Die Mittel sind außerhalb des Strahlengangs des Röntgenstrahlenbündels des Röntgensystems angeordnet und umfassen eine Lichtquelle, eine Fotozelle und einen lichtundurchlässige Marken aufweisenden Ring, welche derart zusammenwirken, daß bei Drehungen des Röntgensystems Lichtimpulse erzeugt werden. Anhand der Lichtimpulse werden die Projektionswinkel während eines Scans für die Bildrekonstruktion ermittelt.

Aus der DE 36 04 955 A1 ist ein Röntgendiagnostikgerät mit einem Bilderzeugungssystem mit Röntgenstrahler und Strahlenempfänger sowie einem Lagerungstisch bekannt. Mit den verstellbaren Komponenten des Bilderzeugungssystems sind Positionsgeber in Form von Potentiometern verbunden, welche die Stellung dieser Komponenten erfassen.

In der DE 195 35 583 A1 ist außerdem ein Röntgendiagnostikgerät mit einer Positionierhilfe beschrieben. An einem Röntgenbildverstärker ist dabei ein Lichtsender zum Aussenden eines Lichtbündels derart vorgesehen, daß dieses auf einen den Röntgenbildverstärker gegenüberliegenden Röntgenstrahler fokussiert ist. Auf diese Weise kann eine Positionierung von Röntgenstrahler und Röntgenbildverstärker in bezug auf ein Untersuchungsobjekt ohne die Verwendung von Röntgenstrahlung erfolgen.

Der Erfindung liegt die Aufgabe zugrunde, eine Röntgeneinrichtung der eingangs genannten Art derart auszuführen, daß die Ermittlung der Projektionsgeometrien vereinfacht ist und die Röntgeneinrichtung sowohl stationär als auch mobil einsetzbar ist.

Nach der Erfindung wird diese Aufgabe gelöst durch eine Röntgeneinrichtung aufweisend ein Röntgengerät mit einem eine Röntgenstrahlenquelle und eine Röntgenstrahlenempfangseinrichtung aufweisenden Röntgensystem, welches zur Aufnahme von 2D-Projektionen aus unterschiedlichen Projektionswinkeln von einem Bereich eines zu untersuchenden Objektes mit anschließender 3D-Bildrekonstruktion des Bereiches des Objektes verstellbar ist, und ein Positionserfassungssystem mit wenigstens einer dem Röntgensystem zugeordneten Kamera, welche den Bewegungen des Röntgensystems folgt, und mit in bezug auf die Kamera ortsfesten, von dem Röntgensystem getrennt angeordneten Mitteln, auf die die Kamera zur Ermittlung der Positionen der Röntgenstrahlenquelle, der Röntgenstrahlenempfangseinrichtung und zur Ermittlung der unterschiedlichen Projektionswinkel der 2D-Projektionen für die 3D-Bildrekonstruktion gerichtet ist, wobei die Kamera und die Mittel außerhalb des von der Röntgenstrahlenquelle ausgehenden Röntgenstrahlenbündels angeordnet sind. Die Erfindung geht von der Überlegung aus, daß die Ermittlung der Projektionsgeometrien durch die Anordnung der Kamera und der Mittel des Positionserfassungssystems derart, daß sie nicht von einem von der Röntgenstrahlenquelle ausgehenden Röntgenstrahlenbündel getroffen werden, gegenüber der bekannten Vorrichtung, welche zur Bestimmung der Projektionsgeometrien von dem Röntgenstrahlenbündel getroffen wird, vereinfacht ist, da die Problematik einer geeigneten auswertbaren Abbildung einer Struktur zur Bestimmung der Projektionsgeometrie in einer 2D-Projektion nicht auftritt. Dadurch wird übrigens auch die Genauigkeit der Bestimmung der Projektionsgeometrien verbessert, da die Ermittlung der Projektionsgeometrien anhand der Kamera und der Mittel nicht wie bei der bekannten Vorrichtung auf der Auswertung kleiner in den 2D-Projektionen abgebildeter Strukturen der Vorrichtung beruht.

Dadurch, daß weder die Kamera noch die Mittel in den 2D-Projektionen abgebildet werden, treten zudem keine Überlagerungen von röntgenpositiven Strukturen zu dem radiologisch zu untersuchenden und darzustellenden Bereich des Objektes in den 2D-Projektionen auf. Auf diese Weise wird auch die Rekonstruktion eines von körperfremden Strukturen freien 3D-Bildes des Lebewesens aus den aufgenommenen 2D-Projektionen vereinfacht. Die Kamera und die Mittel zur Bestimmung der Projektionsgeometrien sind zudem sowohl für mobile als auch stationäre Röntgeneinrichtungen geeignet, wobei mechanische Instabilitäten des Röntgengerätes bei der Verstellung des Röntgensystems für die Bestimmung der Projektionsgeometrien unbedeutend sind.

Die Ermittlung der Projektionswinkel anhand der Auswertung von Kamerabildern also unabhängig von direkten, beispielsweise elektro-mechanischen, oder indirekten, beispielsweise elekto-optischen, Abstandsmessungen bietet zudem den Vorteil, daß Störungen, wie Abnutzungserscheiungen an mechanischen Komponenten bzw. elektrische Störsignale, die exakte Ermittlung der Projektionswinkel nicht beeinträchtigen können.

Gemäß einer besonders bevorzugten Ausführungsform der Erfindung sind an der Röntgenstrahlenquelle und der Röntgenstrahlenempfangseinrichtung mindestens je eine Kamera angeordnet, welche jeweils auf die Mittel gerichtet ist, wobei die Mittel gemäß einer Variante der Erfindung einen Markerring umfassen. Der Markerring unterscheidet sich dabei von dem bekannten Markerring dahingehend, daß er keine röntgenpositiven, zur Ermittlung der Projektionsgeometrien vorgesehenen Strukturen aufweist, wobei der Markerring an sich durchaus aus einem röntgenpositiven Material, z. B. Edelstahl, gefertigt sein kann. Die an der Röntgenstrahlenquelle und der Röntgenstrahlenempfangseinrichtung angeordneten Kameras, welche während der Verstellbewegung des Röntgensystems mitbewegt werden, sind dabei derart auf den ortsfesten Markerring gerichtet, daß anhand der Kameraaufnahmen vom Umfang des Markerrings, welche mit der Aufnahme von 2D-Projektionen synchronisiert sind, die Positionen der Röntgenstrahlenquelle und der Röntgenstrahlenempfangseinrichtung in einem ortsfesten, beispielsweise kartesischen Koordinatensystem, ermittelbar sind. Die Lage des ortsfesten Markerrings in dem ortsfesten Koordinatensystem und die Lage der Kameras relativ zu der Röntgenstrahlenquelle und der Röntgenstrahlenempfangseinrichtung ist dabei bekannt. Anhand der für jede 2D-Projektion ermittelten Position der Röntgenstrahlenquelle und der Röntgenstrahlenempfangseinrichtung in dem Koordinatensystem lassen sich die zugehörigen Projektionswinkel der 2D-Projektionen für die 3D-Bildrekonstruktion ermitteln.

Nach einer Variante der Erfindung ist der Markerring längs seines Umfanges mit definierten, d. h. systematisch erzeugten, Oberflächenstrukturen versehen. Die Aufnahme der Oberflächenstrukturen des Markerrings mittels der Kameras gleichzeitige mit einer Aufnahme einer 2D-Projektion ermöglicht eine eindeutige Identifizierung der Position der Röntgenstrahlenquelle und der Röntgenstrahlenempfangseinrichtung relativ zu dem Markerring und somit die Ermittlung des zu der 2D-Projektion gehörigen Projektionswinkels. Die Grobbestimmung der Projektionswinkel kann im übrigen auch anhand von Positionsgebern des Röntgengerätes erfolgen, an die sich die Feinbestimmung der Projektionswinkel anschließt, in der die von den Kameras aufgenommenen Oberflächenstrukturen des Markerrings analysiert werden. Es genügt dann, die Oberflächenstrukturen des Markerrings in einem Winkelbereich von ca. 30° bis 40° unterschiedlich auszubilden, welche periodisch über den gesamten Umfang des Markerrings wiederholt werden.

Gemäß einer Variante der Erfindung ist der Markerring längs seines Umfanges mit definiert, d. h. systematisch, angeordneten, elektrisch betriebenen optisch aktiven Elementen versehen. Eine weitere Variante der Erfindung sieht vor, daß der Markerring längs seines Umfanges mit definiert angeordneten Infrarotlichtquellen und/oder passiven mit beispielsweise an den Kameras angeordneten Infrarotlichtquellen beleuchteten, das Infrarotlicht reflektierenden Infrarotelementen versehen ist. Auch durch die Aufnahme der definiert angeordneten Elemente und/oder der Infrarotlichtquellen und/oder der beleuchteten Infrarotelemente mittels der Kameras gleichzeitig mit der Aufnahme von 2D-Projektionen ist aus deren Anordnung längs des Umfangs des Markerrings eine eindeutige Bestimmung der Projektionsgeometrien zum Zeitpunkt einer jeden der 2D-Projektionen möglich. Die Art der periodischen Anordnung der Elemente, der Infrarotlichtquellen und der Infrarotelemente längs des Umfanges des Markerrings kann analog zu den Oberflächenstrukturen erfolgen. Die Verwendung von Infrarotlichtquellen setzt im übrigen die Verwendung entsprechender Infrarotkameras voraus.

Eine besonders bevorzugte Ausführungsform der Erfindung sieht vor, daß der Markerring zwei trenn- und zusammenfügbare Ringsegmente aufweist. Dies ist besonders vorteilhaft, wenn gemäß einer weiteren Variante der Erfindung das eine Ringsegment an einer das Objekt aufnehmenden Lagerungsvorrichtung angeordnet ist. In diesem Fall wird das andere Ringsegment immer nur dann auf das eine Ringsegment gesetzt, wenn beabsichtigt ist, eine Serie von 2D-Projektionen von einem Bereich eines auf der Lagerungsvorrichtung angeordneten Objektes anzufertigen. Andernfalls wird das andere Ringsegment entfernt, so daß es sich für andere Untersuchungen nicht störend auswirkt und die Lagerungsvorrichtung frei zugänglich ist.

Die Ermittlung der Projektionswinkel ist im übrigen auch mit an der Röntgenstrahlenquelle und der Röntgenstrahlenempfangseinrichtung relativ zueinander örtlich definiert angeordneten Sende- oder Empfangseinrichtungen möglich, welche mit relativ zueinander örtlich definiert an den Mitteln angeordneten Empfangs- oder Sendeeinrichtungen zusammenwirken. Beispielsweise sind an den ortsfesten Mitteln, welche vorzugsweise einen Markerring umfassen, mehrere örtlich definiert, d. h. deren Lage relativ zueinander ist bekannt, über den Umfang des Markerrings verteilt angebrachte Empfangseinrichtungen und an der Röntgenstrahlenquelle und der Röntgenstrahlenempfangseinrichtung beispielsweise jeweils mindestens zwei Sendeeinrichtungen, welche im Zuge einer Verstellbewegung mit der Röntgenstrahlenquelle und der Röntgenstrahlenempfangseinrichtung mitbewegt werden, relativ zueinander örtlich definiert angeordnet. Zur Bestimmung der Projektionsgeometrien bei verschiedenen 2D-Projektionen senden die Sendeeinrichtungen gleichzeitig mit der Aufnahme einer 2D-Projektion gemäß einer Variante der Erfindung vorzugsweise Schallwellen, z. B. Ultraschallwellen, oder elektromagnetische Wellen, z. B. Lichtwellen oder Mikrowellen, aus, welche die Empfänger empfangen. Die Auswertung der empfangenen Ultraschall-, Licht- oder Mikrowellen ermöglicht anschließend die Bestimmung der genauen Position der Röntgenstrahlenquelle und der Röntgenstrahlenempfangseinrichtung während einer jeden 2D-Projektion, so daß aus den jeweils ermittelten Positionen der Röntgenstrahlenquelle und der Röntgenstrahlenempfangseinrichtung die Projektionswinkel jeder der 2D-Projektionen berechenbar sind. Vorzugsweise werden dabei die Phasen und/oder Laufzeiten der Ultraschall-, Licht- oder Mikrowellen zwischen Sende- und Empfangseinrichtungen ermittelt und ausgewertet.

Ein Ausführungsbeispiel der Erfindung ist in den beigefügten schematischen Zeichnungen dargestellt. Es zeigen:
- FIG 1: eine erfindungsgemäße Röntgeneinrichtung mit einem mobilen C-Bogen-Röntgengerät und einem Positionserfassungssystem,
- FIG 2: die Ansicht in Richtung des Pfeiles II aus FIG 1,
- FIG 3: einen Ausschnitt einer zweiten Ausführungsform der erfindungsgemäßen Röntgeneinrichtung,
- FIG 4: einen Ausschnitt einer dritten Ausführungsform der erfindungsgemäßen Röntgeneinrichtung und
- FIG 5: einen Ausschnitt einer anderen Möglichkeit der Ermittlung von Projektionswinkeln.

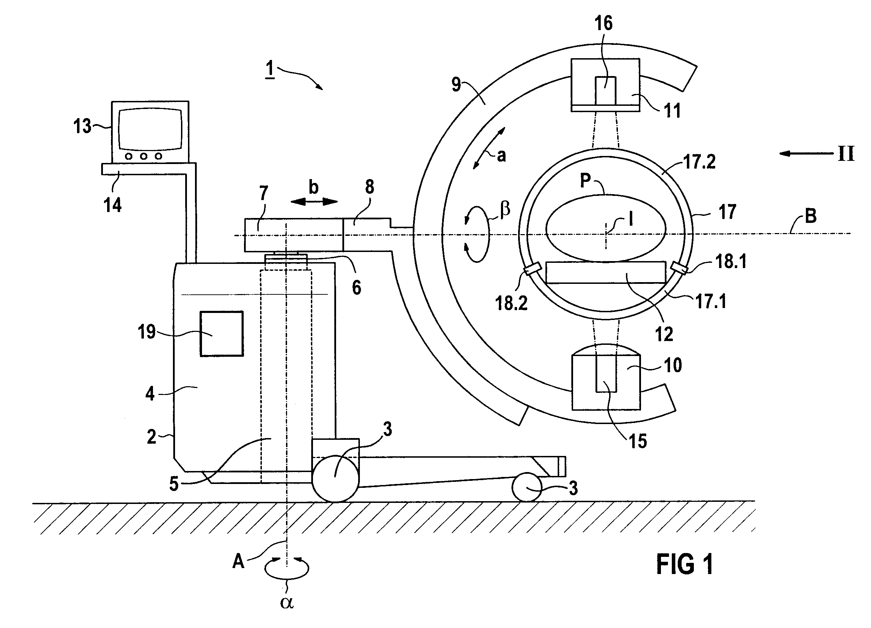

FIG 1 zeigt eine erfindungsgemäße Röntgeneinrichtung 1 mit einem C-Bogen-Röntgengerät 2. Das C-Bogen-Röntgengerät 2 weist einen mit Rädern 3 versehenen Gerätewagen 4 auf. Das C-Bogen-Röntgengerät 2 ist mit einer in FIG 1 nur schematisch angedeuteten Hubvorrichtung 5 mit einer eine Längsachse A aufweisenden Säule 6 versehen, um die die Säule 6 in Richtung des Doppelpfeiles α drehbar ist. An der Säule 6 ist ein Halteteil 7 angeordnet, an dem wiederum ein Lagerteil 8 zur Lagerung eines ein Isozentrum I aufweisenden C-Bogens 9 angeordnet ist. Der C-Bogen 9 weist an seinen beiden Enden einander gegenüberliegend eine Röntgenstrahlenquelle 10 und eine Röntgenstrahlenempfangseinrichtung 11 auf, welche derart relativ zueinander angeordnet sind, daß ein von der Röntgenstrahlenquelle 10 ausgehender Zentralstrahl eines Röntgenstrahlenbündels annähernd mittig auf die Röntgenstrahlenempfangseinrichtung 11 trifft. Der C-Bogen 9 ist in an sich bekannter Weise in Richtung des Doppelpfeiles a längs seines Umfanges in nicht näher dargestellter Weise motorisch verstellbar an dem Lagerteil 8 gelagert. Das Lagerteil 8 ist in an sich bekannter Weise um eine gemeinsame Achse B des Halteteils 7 und des Lagerteils 8 drehbar (vgl. Doppelpfeil β, Angulation) und in Richtung der Achse B verschieblich (vgl. Doppelpfeil b) an dem Halteteil 7 gelagert. Mit Hilfe der Hubvorrichtung 5 ist der C-Bogen 9, der über das Lagerteil 8 und das Halteteil 7 mit der Säule 6 der Hubvorrichtung 5 verbunden ist, relativ zu dem Gerätewagen 4 vertikal verstellbar.

Das C-Bogen-Röntgengerät 2 ist im Falle des vorliegenden Ausführungsbeispiels zur Erzeugung von 3D-Bildern eines Körperbereiches eines in den Figuren nur schematisch dargestellten, auf einer Lagerungsvorrichtung 12 liegenden Patienten P vorgesehen. Die 3D-Bilder werden aus 2D-Projektionen des Körperbereiches aus unterschiedlichen Projektionswinkeln, welche mit Hilfe des die Röntgenstrahlenquelle 10 und die Röntgenstrahlenempfangseinrichtung 11 aufweisenden Röntgensystems gewonnen werden, rekonstruiert und sind mittels eines Monitors 13, welcher auf einem Halter 14 des C-Bogen-Röntgengerätes 2 angeordnet ist, darstellbar.

Zur Aufnahme von 2D-Projektionen aus unterschiedlichen Projektionswinkeln wird der das Röntgensystem aufnehmende C-Bogen 9 längs seines Umfanges in Richtung des Doppelpfeiles a in einem Winkelbereich von ca. 200° um den zu untersuchenden und darzustellenden Körperbereich des Patienten P motorisch verstellt, wobei während der Verstellbewegung ca. 50 - 100 2D-Projektionen von dem Körperbereich des Patienten P mit dem Röntgensystem aus unterschiedlichen Projektionswinkel aufgenommen werden.

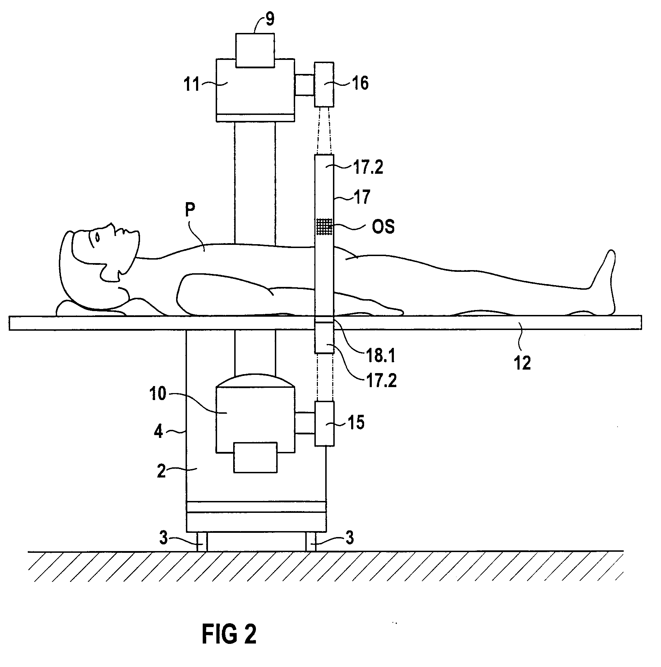

Zur exakten Ermittlung der unterschiedlichen Positionen der Röntgenstrahlenquelle 10, der Röntgenstrahlenempfangseinrichtung 11 und der zugehörigen Projektionswinkel bei aufeinanderfolgenden 2D-Projektionen, welche für die Rekonstruktion von 3D-Bildern des Körperbereiches des Patienten P aus den 2D-Projektionen unbedingt erforderlich sind, ist die Röntgenstrahlenquelle 10 mit einer seitlich angeordneten Kamera 15 (vgl. FIG 2), die Röntgenstrahlenempfangseinrichtung 11 mit einer seitlich angeordneten Kamera 16 (vgl. FIG 2) und die Lagerungsvorrichtung 12 mit einem Markerring 17 versehen. Die Kameras 15, 16 und der Markerring 17 sind Teil eines Positionserfassungssystems.

Der Markerring 17 weist zwei Ringsegmente 17.1 und 17.2 auf, welche an Stellen 18.1, 18.2 zusammenfügbar bzw. voneinander trennbar sind. Das Ringsegment 17.1 ist an in FIG 1 nicht näher dargestellten Schienen, welche an beiden Seiten der Lagerungsvorrichtung 12 vorhanden sind, befestigt und entlang der Schienen verschiebbar. Im Falle der Aufnahme einer Serie von 2D-Projektionen eines Körperbereiches des Patienten P wird das Ringsegment 17.2 mit dem Ringsegment 17.1 an den Stellen 18.1, 18.2 zusammengefügt, so daß der Markerring 17 den Patienten P umgibt. Der Markerring 17 weist über seinen Umfang definierte, d. h. systematisch erzeugte, in FIG 2 nur stark schematisch angedeutete Oberflächenstrukturen OS auf. Anhand der Oberflächenstrukturen OS ist eine eindeutige Identifizierung einer Position in bezug auf den Umfang des Markerrings 17 möglich.

Im Zuge der Aufnahme einer Serie von 2D-Projektionen von einem Körperbereich des Patienten P mit anschließender 3D-Bildrekonstruktion wird das C-Bogen-Röntgengerät 2 entsprechend relativ zu dem zu untersuchenden Körperbereich des Patienten P positioniert. Der Markerring 17 wird derart relativ entlang der Schienen der Lagerungsvorrichtung 12 verschoben, daß die an der Röntgenstrahlenquelle 10 und an der Röntgenstrahlenempfangseinrichtung 11 angeordneten Kameras 15, 16 auf den Umfang des Markerrings 17 gerichtet sind. Auf diese Weise können die Kameras 15, 16 die im Sichtbereich der Kameras 15, 16 liegenden Oberflächenstrukturen OS des Markerrings 17 aufnehmen (vgl. FIG 2 strichpunktierte Linien). Die Lage der Kamera 15 relativ zu der Röntgenstrahlenquelle 10, die Lage der Kamera 16 relativ zu der Röntgenstrahlenempfangseinrichtung 11 und die Lage des Markerrings 17 in einem zur Angabe der Positionen der Röntgenstrahlenquelle 10 und der Röntgenstrahlenempfangseinrichtung 11 vorgesehenen ortsfesten, beispielsweise kartesischen Koordinatensystem, ist dabei bekannt.

Die Aufnahmen der Kameras 15, 16 werden einer Steuer- und Recheneinheit 19 des C-Bogen-Röntgengerätes 2 zugeführt. Die Steuer- und Recheneinheit 19, welche sowohl die motorische Verstellbewegung des C-Bogens 9 steuert als auch die Aufnahme von 2D-Projektionen auslöst, wertet auch die zum Zeitpunkt einer jeden 2D-Projektion aufgenommenen Kamerabilder der Kameras 15, 16 aus. Die Steuer- und Recheneinheit 19 ermittelt anhand der jeweiligen Einstellungen der Kameras 15, 16 und der aufgenommenen Oberflächenstrukturen OS des Markerrings 17 die Abstände der Röntgenstrahlenquelle 10 und der Röntgenstrahlenempfangseinrichtung 11 von und deren Orientierung zu dem Markerring 17. Da der Steuer- und Recheneinheit 19 die Lage des Markerrings 17 in dem kartesischen Koordinatensystem sowie die Lage der Kamera 15 relativ zu der Röntgenstrahlenquelle 10 und die Lage der Kamera 16 relativ zu der Röntgenstrahlenempfangseinrichtung 11 bekannt ist, kann die Steuerund Recheneinheit 19 anhand der ermittelten Abstände und Orientierungen für jede 2D-Projektion die exakte Position der Röntgenstrahlenquelle 10, der Röntgenstrahlenempfangseinrichtung 11 und der zugehörigen Projektionswinkel in bezug auf den Markerring 17 ermitteln. Die ermittelten Positionen werden beispielsweise bezüglich des ortsfesten kartesischen Koordinatensystems angegeben, in der, wie bereits erwähnt, die Lage des Markerrings 17 bekannt ist.

Der Markerring 17 ist vorzugsweise aus einem gegen Verformung stabilen Material, z. B. Edelstahl, gebildet und für Anwendungen in Operationssälen sterilisierbar ausgeführt.

Die Grobbestimmung der Orientierung der Röntgenstrahlenquelle 10 und der Röntgenstrahlenempfangseinrichtung 11 zu dem Markerring 17 erfolgt im übrigen anhand von in der FIG 1 nicht dargestellten, dem C-Bogen 9 zugeordneten Positionsgebern des Röntgengerätes 2, während die Feinbestimmung anhand der Auswertung der Kameraaufnahmen der Oberflächenstrukturen OS des Markerrings 17 erfolgt. Demnach genügt es, die Oberflächenstrukturen OS des Markerrings, beispielsweise nur in einem Winkelbereich von 30° bis 40° verschieden auszuführen. Diese Oberflächenstrukturen OS wiederholen sich dann periodisch über den gesamten Umfang des Markerrings 17. Der Markerring 17 kann aber auch mit Oberflächenstrukturen OS versehen sein, die über den gesamten Umfang des Markerrings 17 voneinander verschieden sind.

Auf diese Weise ist also für jede 2D-Projektion die entsprechende Position der Röntgenstrahlenquelle 10 und der Röntgenstrahlenempfangseinrichtung 11 und somit der jeweilige Projektionswinkel ermittelbar. Die Signalleitungen zur Übertragung der Steuersignale für die motorische Verstellung des C-Bogens 9, die Auslösung von 2D-Projektionen und die Übertragung der Kamerasignale sind in den Figuren im übrigen nicht dargestellt.

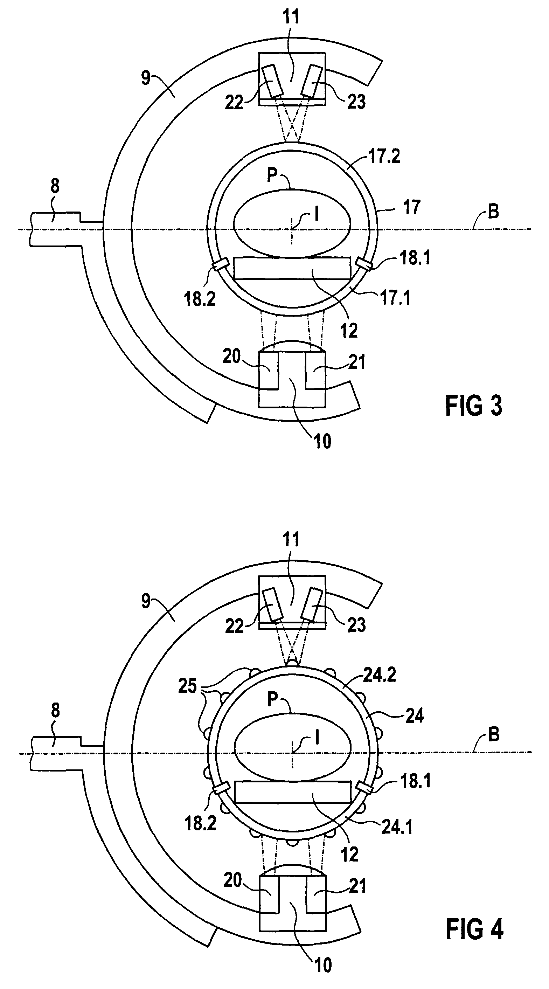

Die FIG 3 zeigt einen Ausschnitt einer zweiten Ausführungsform der erfindungsgemäßen Röntgeneinrichtung, wobei in der FIG 3 dargestellte Komponenten der Röntgeneinrichtung, welche mit Komponenten der in FIG 1 dargestellten Röntgeneinrichtung weitgehend identisch sind, mit gleichen Bezugszeichen versehen sind.

Im Falle dieser Ausführungsform sind die Röntgenstrahlenquelle 10 und die Röntgenstrahlenempfangseinrichtung 11 jeweils mit zwei Kameras versehen. Die Kameras 20, 21 sind an der Röntgenstrahlenquelle 10 derart angeordnet, daß die jeweiligen von den Kameras 20, 21 erfaßten Bereiche des Umfanges des Markerrings 17 möglichst weit voneinander entfernt liegen. Die Kameras 22, 23 sind dagegen derart an der Röntgenstrahlenempfangseinrichtung 11 angeordnet, daß etwa der gleiche Bereich des Umfanges des Markerrings 17 von den Kameras erfaßt wird (photogammetrisches Verfahren). Die Anordnung der Kameras 20 bis 23 an der Röntgenstrahlenquelle 10 und der Röntgenstrahlenempfangseinrichtung 11 ist derart gewählt, daß die Genauigkeit der Positionsbestimmung der Röntgenstrahlenquelle 10, der Röntgenstrahlenempfangseinrichtung 11 und der zugehörigen Projektionswinkel aufgrund der zusätzlich gewonnenen Informationen verbessert ist. Dabei können die Kameras 20, 21 auch wie die Kameras 22, 23 relativ zueinander angeordnet sein. Ebenso können die Kameras 22, 23 wie die Kameras 20, 21 relativ zueinander angeordnet sein.

FIG 4 zeigt einen Ausschnitt einer dritten Ausführungsform der erfindungsgemäßen Röntgeneinrichtung, bei der im Unterschied zu der zweiten Ausführungsform der Röntgeneinrichtung der Markerring 17 durch einen Markerring 24 ersetzt ist. Der Markerring 24 ist längs seines Umfanges mit definiert angeordneten, elektrisch betriebenen optisch aktiven Elementen, die zueinander in einer definierten geometrisch eindeutigen Beziehung stehen, versehen. Bei den optisch aktiven Elementen kann es sich beispielsweise um Leuchtdioden 25 handeln. Wie im zuvor beschriebenen Ausführungsbeispiel nehmen die Kameras 20 bis 23 bei einer Serie von 2D-Projektionen zum Zeitpunkt einer jeden 2D-Projektion die Struktur der optisch aktiven Elemente auf dem Umfang des Markerrings 24 auf. Die in FIG 4 nicht dargestellte Steuer- und Recheneinheit 19 ermittelt wie in zuvor beschriebener Weise für jede 2D-Projektion anhand der jeweiligen Einstellungen der Kameras 20 bis 23 und der aufgenommenen Strukturen der optisch aktiven Elemente auf dem Umfang des Markerrings 24 die Abstände der Röntgenstrahlenquelle 10 und der Röntgenstrahlenempfangseinrichtung 11 von und deren Orientierung zu dem Markerring 24. Da der Steuer- und Recheneinheit 19 wie im zuvor beschriebenen Ausführungsbeispiel die Lage des Markerrings 24 in dem kartesischen Koordinatensystem sowie die Lage der Kameras 20, 21 relativ zu der Röntgenstrahlenquelle 10 und die Lage der Kameras 22, 23 relativ zu der Röntgenstrahlenempfangseinrichtung 11 bekannt ist, kann die Steuer- und Recheneinheit 19 anhand der ermittelten Abstände und Orientierungen für jede 2D-Projektion die genaue Position der Röntgenstrahlenquelle 10, der Röntgenstrahlenempfangseinrichtung 11 sowie die zugehörigen Projektionswinkel in dem kartesischen Koordinatensystem ermitteln. Die derart ermittelten Projektionsgeometrien werden dann zur Rekonstruktion von 3D-Bildern von Körperbereichen des Patienten P mittels der Steuer- und Recheneinheit 19 herangezogen, wobei die 3D-Bilder, wie bereits erwähnt, z. B. auf dem Monitor 13 des C-Bogen-Röntgengerätes 2 darstellbar sind.

Anstelle von Leuchtdioden 25 können im übrigen über den Umfang des Markerrings 24 auch Infrarotlichtquellen und/oder passive mit beispielsweise an den Kameras 20 bis 23 angeordneten Infrarotlichtquellen beleuchtete, das Infrarotlicht reflektierende Infrarotelemente vorgesehen sein. Im Falle der Verwendung von Infrarotlichtquellen sind allerdings anstelle der Kameras 20 bis 23 entsprechende Infrarotkameras zur Aufnahme der Infrarotsignale vorzusehen.

Falls zweckmäßig können elektrisch betriebene optisch aktive Elemente auch mit Infrarotlichtquellen und passiven Infrarotelementen über den Umfang des Markerrings 24 kombiniert werden.

Der Markerring 24 weist im übrigen wie der Markerring 17 zwei an Stellen 18.1 und 18.2 zusammenfügbare bzw. voneinander trennbare Ringsegmente 24.1 und 24.2, wobei das Ringsegment 24.1 an in FIG 4 nicht näher dargestellten Schienen der Lagerungsvorrichtung 12 befestigt ist. Der Markerring 24 ist in nicht dargestellter Weise zur Versorgung der Leuchtdioden 25 mit Energie elektrisch kontaktiert.

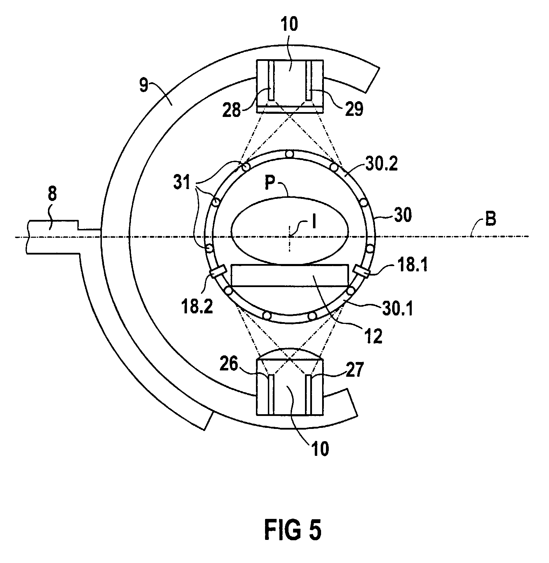

FIG 5 zeigt einen Ausschnitt einer anderen Möglichkeit der Ermittlung von Projektionswinkeln. Im Unterschied zu den zuvor beschriebenen Möglichkeiten sind die an der Röntgenstrahlenquelle 10 und der Röntgenstrahlenempfangseinrichtung 11 angeordneten Kameras durch relativ zueinander örtlich definiert angeordnete Sendeeinrichtungen 26, 27 und 28, 29 und der Markerring 24 durch einen mit relativ zueinander örtlich definiert angeordneten Empfangseinrichtungen 31 versehenen Markerring 30 ersetzt. Im vorliegenden Falle handelt es sich bei den Sendeeinrichtungen um Ultraschallsender 26 bis 29 und bei den Empfangseinrichtungen um Ultraschallempfänger 31. Während die an dem Markerring 30 angeordneten Ultraschallempfänger 31 in bezug auf das Röntgensystem ortsfest sind, werden die Ultraschallsender 26 bis 29 bei der Verstellbewegung des C-Bogens 9 längs seines Umfanges mit dem Röntgensystem mitbewegt.

Die in FIG 5 nicht dargestellte Steuer- und Recheneinheit 19, welche wie im zuvor beschriebenen Ausführungsbeispiel sowohl die motorische Verstellung des C-Bogens 9 steuert als auch die Aufnahme von 2D-Projektionen auslöst, steuert auch die Auslösung von Ultraschallwellen der Ultraschallsender 26 bis 29, welche jeweils gleichzeitig mit der Auslösung einer 2D-Projektion erfolgt. Auf diese Weise ist für jede 2D-Projektion die entsprechende Position der Röntgenstrahlenquelle 10 und der Röntgenstrahlenempfangseinrichtung 11 und somit der jeweilige Projektionswinkel ermittelbar.

Die Ultraschallsender 26 bis 29 werden vorzugsweise mit unterschiedlichen Frequenzen betrieben, so daß die von den Ultraschallempfängern 31 empfangenen Ultraschallwellen eindeutig einem der Ultraschallsender 26 bis 29 zugeordnet werden können.

Senden die Ultraschallsender 26 bis 29 bei der Auslösung einer 2D-Projektion Ultraschallwellen nicht gleichzeitig, sondern in kurzen definierten Zeitabständen hintereinander aus, können die Ultraschallsender 26 bis 29 auch mit der gleichen Sendefrequenz betrieben werden, wobei auch in diesem Fall die von den Ultraschallempfängern 31 empfangenen Ultraschallwellen eindeutig einem der Ultraschallsender 26 bis 29 zugeordnet werden können.

Die Ultraschallsender 26 bis 29 und die Ultraschallempfänger 31 weisen vorzugsweise an die Verstellbewegungen des Röntgensystems angepaßt Richtcharakteristiken auf. Auf diese Weise kann der Empfang von die Signalauswertung störenden Fremdsignalen seitens der Ultraschallempfänger 31 vermindert und die erforderliche Sendeleistung der Ultraschallsender 26 bis 29 gegenüber isotrop strahlenden Ultraschallsendern reduziert werden.
Zur exakten Ermittlung der jeweiligen Positionen der Röntgenstrahlenquelle 10 und der Röntgenstrahlenempfangseinrichtung 11 bei 2D-Projektionen in bezug auf ein ortsfestes, beispielsweise kartesisches Koordinatensystem, sollten vorzugsweise jeweils mindestens drei der Ultraschallempfänger 31 Ultraschallwellen der der Röntgenstrahlenquelle 10 und der Röntgenstrahlenempfangseinrichtung 11 zugeordneten Ultraschallsender 26 bis 29 während der Verstellbewegung des C-Bogens 9 empfangen, d. h. eine beispielsweise von dem Ultraschallsender 26 ausgesandte Ultraschallwelle sollte von mindestens drei Ultraschallempfängern empfangen werden.

Die von den Ultraschallempfängern 31 empfangenen Ultraschallwellen werden anschließend der Steuer- und Recheneinheit 19 zugeführt, welche vorzugsweise die Phasen und/oder die Laufzeiten der jeweiligen zu einer 2D-Projektion gehörigen Ultraschallwellen ermittelt. Da die Laufzeit des Ultraschalls abhängig von der Umgebungstemperatur ist, ist eine in den Figuren nicht dargestellte, an die Steuer- und Recheneinheit 19 angeschlossene Einheit zur Messung der Umgebungstemperatur vorgesehen, welche bei der Ermittlung der Laufzeit des Ultraschalls in Ausgleichsrechnungen berücksichtigt wird.

Anhand der Phasen- und/oder Laufzeitinformationen der empfangenen Ultraschallwellen errechnet die Steuer- und Recheneinheit 19 bei jeder 2D-Projektion für jeden Ultraschallsender 26 bis 29 den Abstand zu den Ultraschallempfängern 31, welche die von den jeweiligen Ultraschallsendern 26 bis 29 ausgesandten Ultraschallwellen empfangen haben. Da der Steuer- und Recheneinheit 19 die Lage des Markerrings 30 bzw. der Ultraschallempfänger 31 in dem kartesischen Koordinatensystem sowie die Lage der Ultraschallsender 26, 27 relativ zu der Röntgenstrahlenquelle 10 und die Lage der Ultraschallsender 28, 29 relativ zu der Röntgenstrahlenempfangseinrichtung 11 bekannt ist, kann die Steuer- und Recheneinheit 19 anhand der ermittelten Abstände für jede 2D-Projektion die genaue Position und die Projektionswinkel der Röntgenstrahlenquelle 10 und der Röntgenstrahlenempfangseinrichtung 11 in bezug auf das ortsfeste Koordinatensystem zu jeder 2D-Projektion exakt ermitteln.

Die Ultraschallsender müssen im übrigen nicht notwendigerweise an der Röntgenstrahlenquelle und der Röntgenstrahlenempfangseinrichtung und die Ultraschallempfänger an dem Markerring angeordnet sein. Vielmehr können die Ultraschallsender auch am Markerring und die Ultraschallempfänger an der Röntgenstrahlenquelle und der Röntgenstrahlenempfangseinrichtung angeordnet sein.

Ist an der Röntgenstrahlenquelle 10 und an der Röntgenstrahlenempfangseinrichtung 11 jeweils nur ein Ultraschallsender vorgesehen, müssen zur exakten Positionsbestimmung jeweils mindestens drei der Ultraschallempfänger 31 Ultraschallwellen der der Röntgenstrahlenquelle 10 und der Röntgenstrahlenempfangseinrichtung 11 zugeordneten Ultraschallsender 26 bis 29 während der Verstellbewegung des C-Bogens 9 empfangen.

Zur exakten Positionsbestimmung der Röntgenstrahlenquelle und des Röntgenstrahlenempfängers können jedoch auch mehr als zwei Ultraschallsender an der Röntgenstrahlenquelle und der Röntgenstrahlenempfangseinrichtung vorgesehen, deren Ultraschallwellen von wenigstens einem der Ultraschallempfänger 31 empfangen werden.

Die Anzahl der zur Positionsbestimmung der Röntgenstrahlenquelle 10 und der Röntgenstrahlenempfangseinrichtung 11 vorgesehenen Ultraschallsender und Ultraschallempfänger kann also von der im vorliegenden Ausführungsbeispiel verwendeten Anzahl abweichen. Des weiteren ist die Anbringung der Ultraschallsender und der Ultraschallempfänger nur exemplarisch zu verstehen und kann auch anders ausgeführt sein.

Im übrigen ist auch der Markerring 30 wie die Markerringe 17, 24 aus zwei trenn- und zusammenfügbaren Ringsegmenten 30.1, 30.2 zusammengesetzt und entsprechend an der Lagerungsvorrichtung 12 angeordnet. Zur Energieversorgung der Ultraschallempfänger 31 und zur Signalübertragung ist der Markerring 30 in nicht dargestellter Weise elektrisch kontaktiert.

Anstelle der Ultraschallsender 26 bis 29 und der Ultraschallempfänger 31 können zur Bestimmung der Projektionsgeometrien der Röntgenstrahlenquelle 10 und der Röntgenstrahlenempfangseinrichtung 11 Sender und Empfänger vorgesehen sein, die auf Basis anderer Schallwellen oder elektromagnetischer Wellen, beispielsweise Licht- oder Mikrowellen, arbeiten.

Die Ermittlung der Projektionsgeometrien beruht auch in diesem Fall auf der Ermittlung von Phasen- und/oder Laufzeiten von Mikrowellen oder Lichtwellen zwischen Sendeeinrichtungen und Empfangseinrichtungen. Anhand der Phasen- und/oder Laufzeitinformationen können wie im Falle der Verwendung von Ultraschall die Abstände zwischen Sende- und Empfangseinrichtungen bestimmt und somit die exakten Positionen und Orientierungen der Röntgenstrahlenquelle und der Röntgenstrahlenempfangseinrichtung relativ zu dem Markerring bei jeder 2D-Projektion ermittelt werden. Im Falle der Anwendung von Lichtwellen zur Bestimmung der Projektionsgeometrien bieten sich als geeignete optische Signalquellen Laserdioden, Leuchtdioden oder Superlumineszenzdioden und als auf dem Markerring angeordnete Detektionsmittel Dioden oder Avalancephotodioden an.

Die erfindungsgemäße Röntgeneinrichtung wurde vorliegend am Beispiel eines mobilen C-Bogen-Röntgengerätes 2 erläutert. Die Verwendung des Positionserfassungssystems zur Positionsbestimmung der Röntgenstrahlenquelle 10 und der Röntgenstrahlenempfangseinrichtung 11 ist jedoch nicht auf den Einsatz in mobilen C-Bogen-Röntgengeräten beschränkt, sondern auch in stationären Röntgengeräten möglich.

## Patentansprüche

1. Röntgeneinrichtung aufweisend ein Röntgengerät (2) mit einem eine Röntgenstrahlenquelle (10) und eine Röntgenstrahlenempfangseinrichtung (11) aufweisenden Röntgensystem, welches zur Aufnahme von 2D-Projektionen aus unterschiedlichen Projektionswinkeln von einem Bereich eines zu untersuchenden Objektes (P) mit anschließender 3D-Bildrekonstruktion des Bereiches des Objektes (P) verstellbar ist, und ein Positionserfassungssystem mit wenigstens einer dem Röntgensystem zugeordneten Kamera (15, 16, 20 bis 23), welche den Bewegungen des Röntgensystems folgt, und mit in bezug auf die Kamera (15, 16, 20 bis 23) ortsfesten, von dem Röntgensystem getrennt angeordneten Mitteln (17, 24), auf die die Kamera (15, 16, 20 bis 23) zur Ermittlung der Positionen der Röntgenstrahlenquelle (10), der Röntgenstrahlenempfangseinrichtung (11) und zur Ermittlung der unterschiedlichen Projektionswinkel der 2D-Projektionen für die 3D-Bildrekonstruktion gerichtet ist, wobei die Kamera (15, 16, 20 bis 23) und die (17, 24) Mittel außerhalb des von der Röntgenstrahlenquelle (10) ausgehenden Röntgenstrahlenbündels angeordnet sind.

2. Röntgeneinrichtung nach Anspruch 1, bei der an der Röntgenstrahlenquelle (10) und der Röntgenstrahlenempfangseinrichtung (11) mindestens je eine, auf die Mittel (17, 24) gerichtete Kamera (15, 16, 20 bis 23) angeordnete ist.

3. Röntgeneinrichtung nach einem der Ansprüche 1 oder 2, bei der die Mittel einen Markerring (17, 24) umfassen.

4. Röntgeneinrichtung nach Anspruch 3, bei der der Markerring (17) längs seines Umfanges mit definierten Oberflächenstrukturen versehen ist.

5. Röntgeneinrichtung nach einem der Ansprüche 3 oder 4, bei der der Markerring (24) längs seines Umfanges mit definiert angeordneten, elektrisch betriebenen optisch aktiven Elementen (25) versehen ist.

6. Röntgeneinrichtung nach einem der Ansprüche 3 bis 5, bei der der Markerring (24) längs seines Umfanges mit definiert angeordneten Infrarotlichtquellen und/oder passiven mit Infrarotlichtquellen beleuchteten Infrarotelementen versehen ist.

7. Röntgeneinrichtung nach einem der Ansprüche 3 bis 6, bei der der Markerring (17, 24) zwei trenn- und zusammenfügbare Ringsegmente (17.1, 17.2, 24.1, 24.2) aufweist.

8. Röntgeneinrichtung nach Anspruch 7, bei der das eine Ringsegment (17.1, 24.1) an einer das Objekt (P) aufnehmenden Lagerungsvorrichtung (12) angeordnet ist.
